# EUROPÄISCHE PATENTSCHRIFT

(11) **EP 1 324 094 B1**
(45) Veröffentlichungstag und Bekanntmachung des Hinweises auf die Patenterteilung: **26.03.2008**
(21) Anmeldenummer: 02102643.0
(22) Anmeldetag: 26.11.2002
(51) Int. Cl.: G02B 21/00, F16M 11/04, G02B 23/16, G02B 7/00

(54) **Stativ mit Operationsmikroskop**
Support with a surgical microscope
Statif avec un microscope chirurgical

(30) Priorität: 21.12.2001 DE 10163354
(43) Veröffentlichungstag der Anmeldung: 02.07.2003
(73) Patentinhaber: Leica Microsystems (Schweiz) AG, 9435 Heerbrugg (CH)
(72) Erfinder: METELSKI, Andrzej, 8590, Romanshorn (CH)
(74) Vertreter: Rosenich, Paul

(56) Entgegenhaltungen:
- EP-A- 0 592 703
- EP-A- 0 593 791
- WO-A-01/40700
- DE-U- 8 024 962
- US-A- 4 852 842
- PATENT ABSTRACTS OF JAPAN vol. 1998, no. 10, 31. August 1998 (1998-08-31) -& JP 10 122481 A (SANSHIYUUZEN KOGYO KK;KAJIWARA KOJI), 15. Mai 1998 (1998-05-15)

## Beschreibung

Die Erfindung betrifft eine Vorrichtung zum Halten eines Operationsmikroskops mit parallelogrammartig angeordneten beweglichen Halteträgern mit einem diagonalen Stützelement. Unter Stützelement sind beispielsweise Diagonalstreben oder dergleichen zu verstehen, die in einer mechanisch-geometrischen Anordnung Kräfte übertragen und damit dem Abstützen bzw. Halten und/oder Lageverändern der Betrachtungseinrichtung dienen können. Sie wirken dabei nach Hebel- und/oder Momentgesetzen und beinhalten gegebenenfalls Verstellvorrichtungen, um geänderten Verhältnissen insofern Rechnung zu tragen oder geänderte Verhältnisse herbeiführen, so dass das Halten und/oder Lageverändern der Betrachtungseinrichtung gewährleistet werden kann.

Die Vorrichtung soll auf einem Stativfuss beziehungsweise auf einer Stativsäule wie ein Stativarm verwendet werden können, aber auch an anderen Stativen wie Decken- oder Wandstativen einsetzbar sein.

Das Halten einer optischen Betrachtungseinrichtung mittels eines Stativs mit Stativarm, wird bei verschiedensten Anwendungen, insbesondere im Bereich der Medizinaltechnik, benötigt. Häufig werden diese Stativarme als einarmige Träger ausgebildet, welche mittels Federmechanismen und/oder feststellbaren Bremsen in ihrer Position gehalten werden und/oder motorisch angetrieben sind.

In der WO-99/01693-A ist ein Stativ dargestellt, das von der Anmelderin als MS 1 vertrieben wird. Es verfügt, wie schon Vorgängermodelle, über einen als Parallelogramm aufgebauten Tragarm, der durch eine schräg angeordnete Stützfeder in seiner geometrischen Erstreckung definiert ist, wobei die Stützfeder als Gewichtskompensation für die Last der Betrachtungseinrichtung dient. Die Federkraft ist bei diesem bekannten Aufbau einstellbar, so dass unterschiedliche Gewichte unterschiedlicher oder unterschiedlich ausgerüsteter Betrachtungseinrichtungen aufgenommen bzw. kompensiert werden können.

Die EP-866260-A offenbart ein zahnriemengestütztes Stützelement, das durch seine geometrische Anordnung die Kräfte für den Gewichtsausgleich (Balance) überträgt.

Gebrauchsmuster DE 8 024 962 offenbart ebenfalls ein verstellbares Stativ für ein Operationsmikroskop.

Diagonalstreben werden bei herkömmlichen Aufbauten somit grundsätzlich dazu verwendet, um bestimmt angeordnete Tragarme bzw. Tragarmteile in einer gewünschten Position zu unterstützen bzw. die Last an diesen Aufbauten schräg in den Stativständer einzuleiten. Diese Aufgabe könnte bei diesen Aufbauten zur Unterstützungs-Funktionserfüllung unter Umständen auch dann gelöst sein, wenn gar kein Parallelogramm vorliegt, sondern der Gesamtaufbau galgenförmig ist.

Herkömmliche Diagonalstreben verfügen häufig auch über:
- eine integrierter Federkraft einer Gasdruckfeder ― wie schon oben angegeben- zur (Gewichts-) Kompensation für die Last.
- ein oder mehrere Dämpfungselemente, beispielsweise Gasfedern oder spezielle Gummipuffer mit vorgegebener Feder- und Dämpfungscharakteristik (Schwingungsdämpfung).
- Flüssigkeitsdämpfer.

Der Erfinder erkannte, dass die bekannten Systeme nachteilig sind in Bezug auf die folgenden Punkte:
a) Mangelnde Stabilität in vertikaler Richtung des Mikroskops (parallel zur optischen Achse) und dadurch geringe Positionstreue. Infolge schlecht kontrollierbarer Schwingungs-Stabilität kann es bei einem langsam reagierenden Autofokus zu unscharfen bzw. unstabilen Bildern kommen.
b) Schwere Bauart, um eine verbesserte Stabilität und Schwingungsarmut zu erreichen.
c) Es sind hohe Haltekräfte erforderlich. Die Elemente bauen dadurch massiv, wodurch es - bedingt durch das erhöhte Gewicht - auch zu Trägheit und mangelnder Beweglichkeit kommt.

Der Erfindung liegt somit die Aufgabe zugrunde, eine Vorrichtung zu schaffen, welche das stabile, schwingungsfreie und reproduzierbare Halten einer optischen Betrachtungseinrichtung ermöglicht und/oder die oben angegebenen Nachteile vermeidet. Insbesondere soll in vertikaler Richtung ein verbessertes Schwingungsverhalten auftreten. Durch die mechanischen Eigenschaften des Aufbaus soll auch eine höhere Festigkeit des Gesamtaufbaus gegeben sein. Das Gewicht des Gesamtaufbaus soll möglichst klein gehalten werden.

Gelöst wird diese Aufgabe durch eine Vorrichtung zum Halten einer optischen Betrachtungseinrichtung, beispielsweise eines Mikroskops, auf einem Stativ mit wenigstens einem Stützelement, das Stativteile zueinander abstützt, wobei das Stützelement so angeordnet oder ausgerüstet ist, dass es im Betriebszustand zu einer Verspannung bzw Versteifung von tragenden Stativteilen, insbesondere einer parallelogrammartigen Trägerkonstruktion kommt. Das Abstützelement greift erfindungsgemäss so in die geometrischen oder physikalischen Eigenschaften eines Tragarms - insbesondere eines Parallelogramms - ein, dass dieses verspannt bzw. mechanisch überbestimmt wird.

Durch diesen Erfindungsgedanken sollen Ausführungsformen nicht ausgenommen sein, die ferngesteuert so verstellt werden kann, dass sich die geometriebedingten Kraftwirkungen bzw. Stütz- bzw. Dämpfwirkungen des Abstützelements bedarfsgemäss verändern. Ebenso bedeutet Verspannung bzw. Überbestimmung im Sinne der Erfindung, dass das Stützelement auch in gewissen jedoch engen Bereichen dämpfend nachgeben könnte oder federnd wirken kann.

Entscheidend für die Erzielung der erfindungsgemässen Wirkung ist eine gezielte und willkürlich herbeigeführte Verspannung von Stativteilen zueinander.

Die Bedeutung "Stützelement" im Sinne der Erfindung umfasst auch mehrere z.B. neben einander angeordnete Stützelemente, die gemeinsam wirken.

Bei einer häufig einzusetzende erfindungsgemässen Vorrichtung wird das Stützelement etwa diagonal in einem aus vier Trägern bestehenden Tragarm (Parallelogramm) eingesetzt, bei dem drei der Träger relativ zum vierten und/oder zueinander schwenkbar sind.

Gemäss der Erfindung ist das Stützelement motorisch längenveränderbar, wobei für die Längenveränderung vorzugsweise wenigstens ein fernsteuerbarer Antrieb vorgesehen ist.

Der Antrieb kann ausserhalb des Stützelements oder auch innerhalb desselben angeordnet sein und sowohl elektrische als auch pneumatische Motore umfassen. Insbesondere sind Spindeltriebe, Teleskopelemente oder dergleichen und deren Äquivalente umfasst.

Darüber hinaus ist das Stützelement - beispielsweise durch den Anwender - längenveränderbar und in jeder Längeneinstellung vorzugsweise durch eine - insbesondere fernsteuerbare - Bremse blockierbar. Dies kann so ausgeführt sein, dass das Stützelement wie ein ausziehbares Teleskoprohr als Diagonalstrebe in einem Parallelogramm angeordnet ist und in der Grundstellung durch eine - die Teleskopelemente zueinander blockierende - Bremse blockiert ist.

Besonders für stereotaktische Operationen oder Vorführungen ist es vorteilhaft, wenn eine kombinierte Bewegungs-/Positions-Erfassungseinheit oder eine Positions-Erfassungseinheit und jeweils eine Positions-Speichereinheit vorgesehen sind, die eine wiederholbare Positionierung auf vorgegebene oder gewählte Positionen ermöglicht.

Einen hohen Automatisierungsgrad kann man erreichen, wenn die Bewegungs-/Positions-Erfassungseinheit und/oder die Positions-Speichereinheit, wenigstens eine Prozessorsteuerung zur automatischen Steuerung der Verschiebung bzw. Positionierung umfasst.

Solche Steuerungen können dann auch anwenderspezifisch eingesetzt werden, indem die Prozesssteuerung gegebenenfalls die jeweilige Lage, Neigung usw. des Trägers erfasst und danach die Antriebe steuert, um für einen bestimmten Chirurgen eine bestimmte Stellung einzunehmen. Zur Verbesserung des Dämpfungsverhaltens kann das Stützelement mit einem Dämpfungselement ausgestattet sein, das in Serie oder parallel zum eigentlichen Stützelement (z.B.: einer Diagonalstrebe) angeordnet ist.

Gemäss einer Weiterentwicklung kann die Erfindung auch Bewegungs-, Positions-, Winkel- oder Schwingungssensoren umfassen, die es erlauben, das Stativ in Verbindung mit einer Steuerung oder in einem IGS (Image Guided Surgery) System zu integrieren und computergesteuert zu benutzen.

Eine besondere Präzision in der Anwendung der Erfindung lässt sich erreichen, wenn das Stützelement in seinen Anlenkpunkten und relativ zu seinen Antrieben spielfrei gehalten ist.

Es lassen sich alle erwähnten Varianten beliebig kombinieren, um optimale zielorientierte Ergebnisse zu erreichen.

Durch den vorgängig beschriebenen Einsatz einer erfindungsgemässen Vorrichtung werden weiters noch folgenden Verbesserungen erreicht:
- Hohe Stabilität bei leichter Bauart.
- Schwingungsarmut trotz leichter Bauart.
- Bei den Varianten mit Antrieb (mit oder ohne Bremse): Vorgebbare und ansteuerbare Neigungswinkel bzw. Vertikalpositionen der optischen Betrachtungseinrichtung.
- Bei den Varianten mit elektronischer Steuerung die Möglichkeit der Kombination mit einem Autofokus-System des Mikroskops.
- Konstruktiv einfache Lösung, nachrüstfähig und wirkungsvoll

Die Bezugszeichenliste und die Zeichnungen sind zusammen mit den in den Ansprüchen beschriebenen Gegenständen integrierender Bestandteil der Offenbarung dieser Anmeldung.

An Hand der schematischen Zeichnung wird die Erfindung an verschiedenen Beispielen dargestellt. Es zeigen dabei:
- Fig.1: einen schematischen Ausschnitt aus einem herkömmlichen Stativ mit einem Parallelogrammträger mit längenverstellbarer Diagonalstrebe; das nicht Gegenstand der Erfindung ist;
- Fig.2: einen schematischen Gesamtaufbau eines Stativs mit einem nach Art eines Parallelogramms aufgebauten Stativarm mit gebremster Diagonalstrebe, einem Ausgleichsgewicht und einem Ständer; das nicht Gegenstand der Erfindung ist;

- Fig.3: den Aufbau nach Fig.1 mit längenverstellbarer Diagonalstrebe und einer Bewegungs-/Positions-Erfassungseinheit sowie elektronischer Ansteuerung für den Diagonalstreben-Antrieb; das nicht Gegenstand der Erfindung ist;
- Fig.4: den erfindungsgemäßen Aufbau nach Fig.3 mit motorisch angetriebener Diagonalstrebe und einer davon unabhängig wirkenden zweiten Diagonalstrebe mit Bremse zur motorischen Versteifung und Joystickansteuerung für verschiedenste Steuerungsvarianten;
- Fig.5: eine erfindungsgemäße Variante zum Aufbau gem. Fig.4 mit elektronischer Steuerung und Bewegungs-/Positions-Erfassungseinheit und
- Fig.6: eine Variante mit einer Spindel innerhalb des Parallelogramms, die nicht Gegenstand der Erfindung ist;

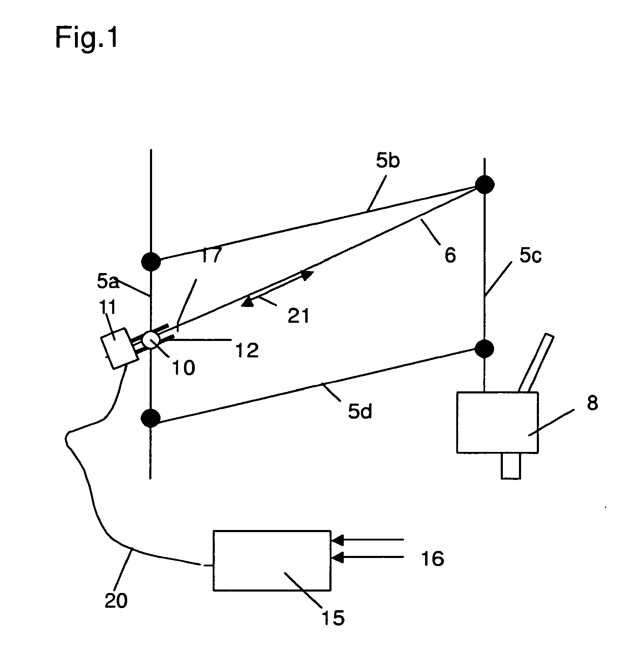

Fig.1 zeigt einen schematischen Ausschnitt aus einem herkömmlichen Stativ mit einem Parallelogrammträger 5a-5d und einer längenverstellbaren Diagonalstrebe 6. Die Diagonalstrebe 6 versteift den Parallelogrammträger 5a-5d, indem sie unter Kraft von dem Träger 5a in das Lager zwischen den Trägern 5b und 5c - durch einen Motor 11 vorgespannt - drückt. Sie verfügt im unteren Bereich über eine Spindel 17, die in einem Gewinde 12 einer Gelenklagerung 10 an dem Halteträger 5a drehgelagert ist. Ein Doppelpfeil 21 deutet die axiale Kraft bzw. Verschiebung der Diagonalstrebe 6 an.

Das Gewicht eines Mikroskops 8 wird somit von dem - mittels Diagonalstrebe 6 versteiften - Parallelogramm 5a-5d aufgenommen. Eine Steuerung 15, in die von Bedienerseite ein Input 16 eingegeben werden kann, steuert den Motor 11 über eine Steuerleitung 20.

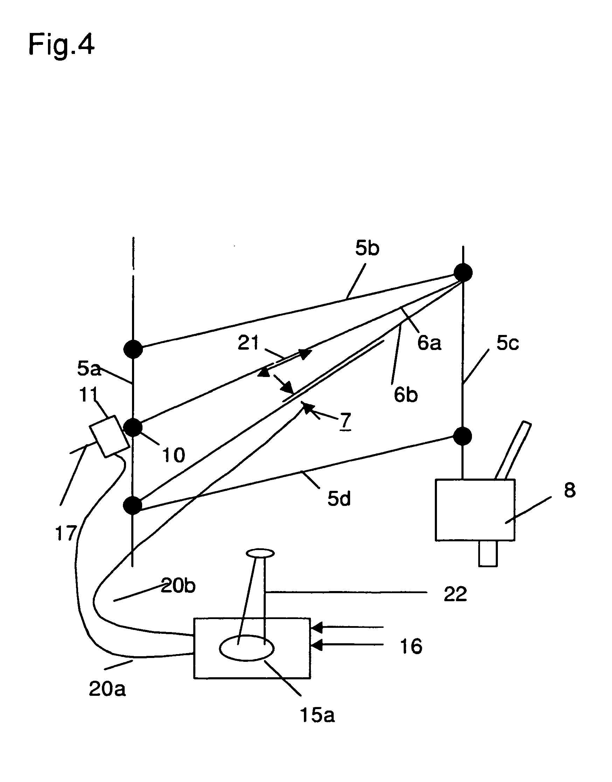

Wie aus Fig.4 ersichtlich, kann eine solche Steuerung 15a z.B. mit einem Joystick 22 oder dergleichen kombiniert werden, um eine leicht bedienbare Handhabung zu ermöglichen.

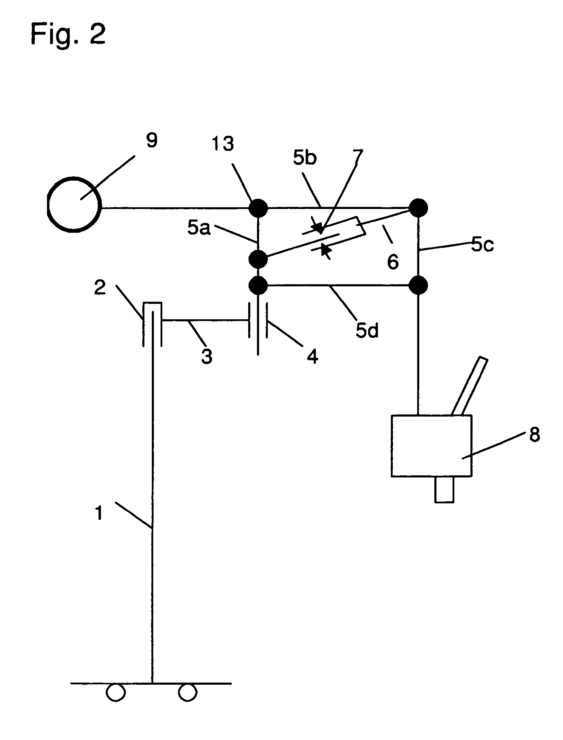

Fig.2 zeigt den schematischen Aufbau eines Stativs für ein Operationsmikroskop 8 mit einem Ständer 1, einem Drehgelenk 2 für einen Stativarm 3, einem Drehgelenk 4 für die vier nach Art eines Parallelogramms angeordneten beweglichen Halteträgern 5a-5d und eine erfindungsgemässe Diagonalstrebe 6 mit einer Bremse 7, die im gelösten Zustand eine Vertikalbewegung des Halteträgers 5c erlaubt.

Der vertikale Halteträger 5a ist in vertikaler Richtung nicht beweglich. Gegebenenfalls ist sie im Lager 4 drehbar. Im eingebremsten Zustand wird das Heben oder Senken der Träger 5b,5c und 5d verhindert.

Die Bremse 7 ist nur symbolisch vereinfacht dargestellt. In den meisten Anwendungsfällen wird sie über elektrisch angesteuerte Bremsbacken verfügen, die Teile der Diagonalstrebe 6 zueinander einbremsen. Dabei kann ein gewisser Reibungseffekt bis zur endgültigen Einbremsung beabsichtigt sein, um so eine Reibungsdämpfung zu erzielen. Dieser Dämpfungsvorgang wirkt natürlich nur so lange, so lange die Bremse nicht im versteiften Zustand des Parallelogramms 5 angezogen ist. Im versteiften Zustand sollte normalerweise keine Bewegung stattfinden. In aussergewöhnlichen Belastungszuständen könnte - im Rahmen einer besonderen Ausbildung der Erfindung - dennoch eine Dämpfungswirkung bzw. ein- wenigstens geringes - Nachgeben der Diagonalstrebe 6 im Sinne einer Überlastsicherheit wirksam werden.

Ein Ausgleichsgewicht 9 balanciert über ein Lager 13 das Gewicht des Mikroskops 8 und der Halteträger 5b-d. Die Bremse 7 hat somit keine explizite Haltefunktion sondern nur eine Versteifungsfunktion und gegebenenfalls eine Schwingungsdämpfungsfunktion für das Parallelogramm.

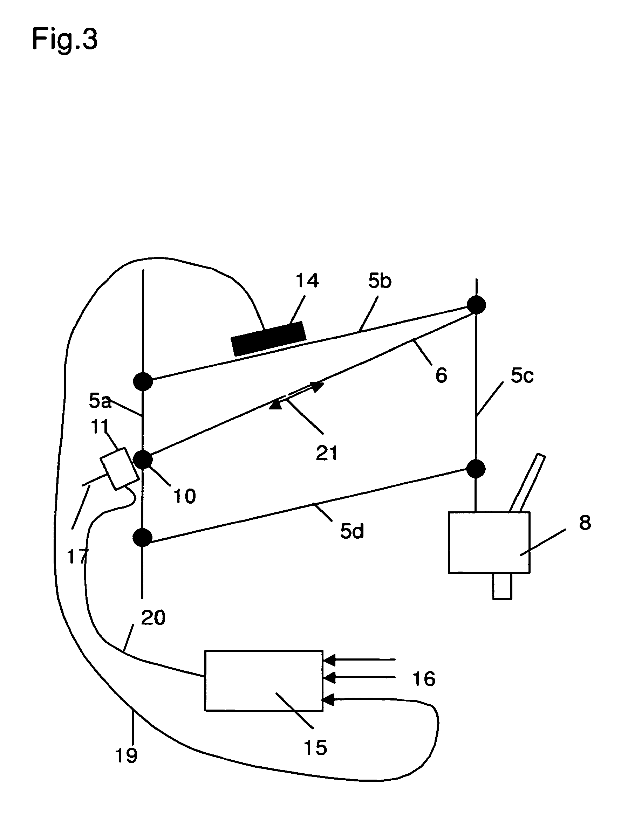

Fig.3 zeigt schematisch einen Stativarm gemäss Fig.1, jedoch mit einer Bewegungs-/Positions-Erfassungseinheit 14, der mit dem Träger 5b verbunden ist, jedoch auch an den anderen Trägern 5c, 5d oder 6 angeordnet sein kann. Die Bewegungs-/Positions-Erfassungseinheit 14 ist in ihrer Darstellung nur ein Symbol, das auch mehrere Bewegungs-/Positions-Erfassungseinheiten oder dergleichen darstellen könnte.

Mit einem geeigneten Programm ausgestattet wird die elektronische Steuereinheit 15, welche beispielsweise nebst den Inputs 16 des Benutzers auch durch die Bewegungs-/Positions-Erfassungseinheit 14 über eine Messleitung 19 mit Informationen versorgt wird, zur Ansteuerung des Motors 11 über die Steuerleitung 20, eingesetzt.

Die Doppelpfeile 21 deuten die Verlängerbarkeit der Diagonalstrebe 6 innerhalb des Parallelogramms 5 an.

Unter "motorisch angetrieben" sind Aufbauten mit beliebigen motorischen Antrieben wie elektrische, elektromotorische, hydraulische, pneumatische oder ähnliche zu verstehen.

Beim erfindungsgemäßen Aufbau nach Fig.4 sieht man eine "Doppeldiagonalstrebe" - symbolisch aus einer ersten Diagonalstrebe 6a und einer zweiten Diagonalstrebe 6b, die gemeinsam mit der ersten Diagonalstrebe 6a die Stützfunktion übernimmt - dargestellt. Die beiden Diagonalstreben 6a und 6b haben unterschiedliche Aufgaben. Während die eine 6a die bereits in den Figuren 1 und 3 beschriebene Aufgabe hat, hat die andere 6b etwa die Aufgabe der Bremse 7 gemäss Fig.3, so dass sich beide in ihrer Wirkung ergänzen.

Ein symbolisch dargestellter Joystick 22 erlaubt das Ansteuern der Bremse 7 und des Motors 11 über die Steuerleitungen 20a und 20b.

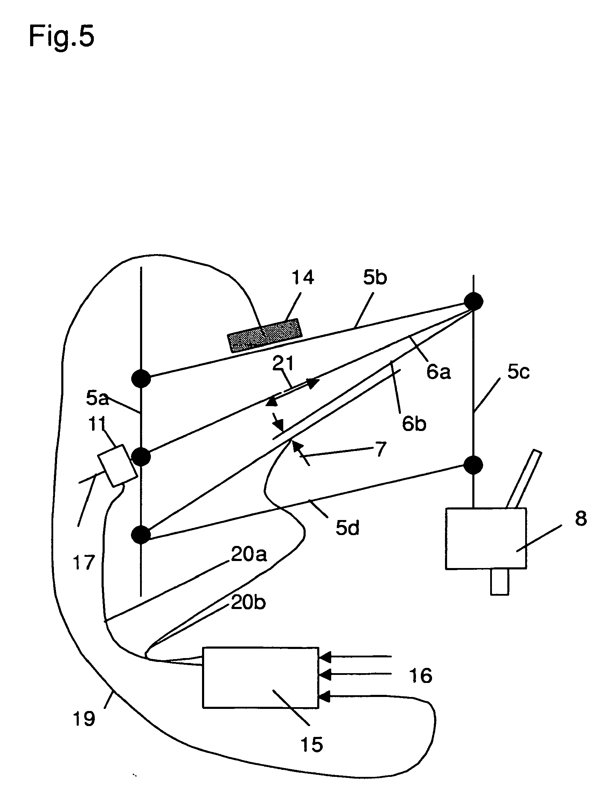

Der erfindungsgemäße Aufbau nach Fig.5 ist dazu noch insofern unterschiedlich, als zusätzlich eine Bewegungs-/Positions-Erfassungseinheit 14 vorgesehen ist, die etwa den oben angegebenen Bewegungs-/Positions-Erfassungseinheiten 14 entspricht und eine selbsttätige Positionierung der Träger 5b,5c und 5d erlaubt, sofern das Programm in der Steuerung entsprechend ausgerüstet ist. Die Steuerleitungen 20a und 20b dienen der Übertragung der Steuerinformation von der Steuerung 15 zu dem Motor 11 und der Bremse 7.

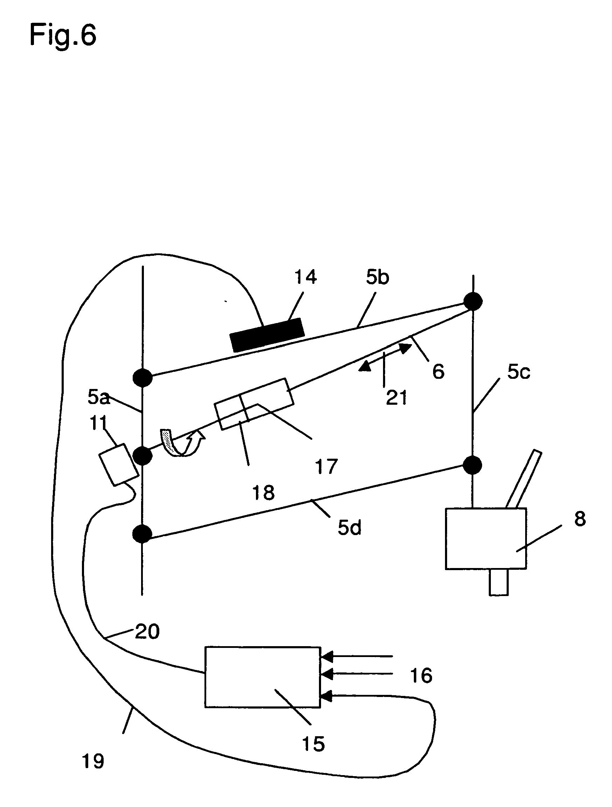

Der Aufbau nach Fig.6 zeigt einen Motor 11, der eine Spindel 17 dreht, die in einer Mutter 18 an der Diagonalstrebe gelagert ist und so eine Längenvariation der Diagonalstrebe 6 ermöglicht.

### Bezugszeichenliste

- 1: Ständer bzw. Stativfuss
- 2: Drehgelenk Stativfuss
- 3: Stativarm
- 4: Drehgelenk Stativarm
- 5: a,b,c,d Nach Art eines Parallelogramms angeordnete bewegliche Halteträger bzw. Träger bzw. Strebe
- 6: Diagonalstrebe 6a, 6b (Stützelement)
- 7: Bremse
- 8: Mikroskop
- 9: Ausgleichsgewicht
- 10: Gelenklager
- 11: Motor
- 12: Gewinde
- 13: Lager
- 14: Bewegungs-/Positions-Erfassungseinheit für Bewegung und/oder Schwingung und/oder Position oder dergleichen
- 15: Steuerung; 15a Steuerung mit Joystick
- 16: Input-Signale
- 17: Spindel
- 18: Mutter
- 19: Messleitung
- 20: a,b, Steuerleitung
- 21: Doppelpfeil
- 22: Joystick

## Patentansprüche

1. Stativ mit einem Operationsmikroskop (8) mit wenigstens einem Stützelement (6, 6a, 6b), das eine Trägerkonstruktion aus parallelogrammartigen Stativteilen zueinander abstützt, wobei die Trägerkonstruktion aus vier zueinander schwenkbaren Trägern (5a-5d) mit einem Lager zwischen zwei benachbarten Trägern (5b, 5c) aufgebaut ist, **dadurch gekennzeichnet, dass** das Stützelement (6, 6a, 6b) als erste und zweite Diagonalstrebe ausgebildet und etwa diagonal in die Trägerkonstruktion eingesetzt ist und direkt in das Lager drückt, so dass die Trägerkonstruktion versteift wird, wobei die erste Diagonalstrebe (6, 6a, 6b) motorisch längenveränderbar ist und die zweite unabhängig wirkende Diagonalstrebe in jeder Längenstellung durch eine fernsteuerbare Bremse (7) blockierbar ausgebildet ist.

2. Stativ nach Anspruch 1, **dadurch gekennzeichnet, dass** für die Längenveränderung wenigstens ein fernsteuerbarer Antrieb bereitgestellt ist und der Antrieb außerhalb der Diagonalstrebe des Stützelements (6, 6a, 6b) angeordnet ist und einen Spindeltrieb (17) umfasst.

3. Stativ nach einem der vorhergehenden Ansprüche, **dadurch gekennzeichnet, dass** der ersten Diagonalstrebe (6, 6a, 6b) für die Längenveränderung wenigstens einen Motor (11) aufweist, der elektrisch oder hydraulisch oder pneumatisch oder als Federmotor ausgebildet ist.

4. Stativ nach einem der vorhergehenden Ansprüche, **dadurch gekennzeichnet, dass** eine Bewegungs-/Positions-Erfassungseinheit (14) und eine Positions-Speichereinheit bereitgestellt sind, die eine wiederholbare Positionierung auf vorgegebene Positionen ermöglicht und die Bewegungs-/Positions-Erfassungseinheit (14) und/oder die Positions-Speichereinheit, wenigstens eine Prozessorsteuerung (15) zur automatischen Steuerung der Verschiebung oder Positionierung umfasst.

5. Stativ nach einem der vorhergehenden Ansprüche, **dadurch gekennzeichnet, dass** die zweite Diagonalstrebe (6, 6a, 6b) mit einem Dämpfungselement ausgestattet ist.

6. Stativ nach einem der vorhergehenden Ansprüche, **dadurch gekennzeichnet, dass** sie es Bewegungs-, Positions-, Winkel- oder Schwingungssensoren (14) umfasst.

7. Stativ nach einem der Ansprüche 2-6, **dadurch gekennzeichnet dass** die Diagonalstrebe (6, 6a, 6b) in ihren Anlenkpunkten innerhalb der Trägerkonstruktion mit vier zueinander schwenkbaren Trägern (5a-5d) und relativ zu ihren Antrieben (11) spielfrei gehalten ist.

## Claims

1. Stand with a surgical microscope (8), with at least one support element (6, 6a, 6b) supporting a carrier construction composed of parallelogram-type stand parts in relation to one another, wherein the carrier construction is made up of four carriers (5a-5d), which can pivot in relation to one another, with a bearing between two neighbouring carriers (5b, 5c), **characterized in that** the support element (6, 6a, 6b) is designed as a first and second diagonal strut and is inserted into the carrier construction approximately diagonally and pushes directly into the bearing such that the carrier construction is stiffened, wherein the length of the first diagonal strut (6, 6a, 6b) can be changed by means of a motor and the second, independently acting diagonal strut is designed such that it can be locked at every length setting by a remote-controlled brake (7).

2. Stand according to Claim 1, **characterized in that** for changing the length, at least one remote-controlled drive is provided and the drive is arranged outside the diagonal strut of the support element (6, 6a, 6b) and comprises a spindle drive (17).

3. Stand according to one of the preceding claims, **characterized in that** the first diagonal strut (6, 6a, 6b) has at least one motor (11) for changing the length, which motor is designed to be electric or hydraulic or pneumatic or as a spring motor.

4. Stand according to one of the preceding claims, **characterized in that** a movement/position detection unit (14) and a position memory unit are provided which enables a reproducible positioning at prespecified positions, and the movement/position detection unit (14) and/or the position memory unit comprises at least one processor control (15) for the automatic control of the displacement or positioning.

5. Stand according to one of the preceding claims, **characterized in that** the second diagonal strut (6, 6a, 6b) has a damping element fitted.

6. Stand according to one of the preceding claims, **characterized in that** it comprises movement, position, angle or oscillation sensors (14).

7. Stand according to one of Claims 2-6, **characterized in that** the diagonal strut (6, 6a, 6b) in its articulation points is held within the carrier construction with four carriers (5a-5d), which can pivot in relation to one another, in a play-free manner in relation to its drives (11).

## Revendications

1. Statif avec un microscope chirurgical (8) comprenant au moins un élément de support (6, 6a, 6b) qui supporte une construction porteuse constituée de pièces de statif en forme de parallélogramme, la construction porteuse se composant de quatre supports pouvant pivoter les uns par rapport aux autres (5a-5d) avec un palier entre deux supports adjacents (5b, 5c), **caractérisé en ce que** l'élément de support (6, 6a, 6b) est réalisé sous forme de premier et deuxième montants diagonaux et est inséré approximativement en diagonale dans la construction porteuse, et presse directement dans le palier, de manière à rigidifier la construction porteuse, la longueur du premier montant diagonal (6, 6a, 6b) pouvant être modifiée de manière motorisée et le deuxième montant diagonal agissant indépendamment étant réalisé de manière à pouvoir être bloqué dans chaque position en longueur par un frein (7) télécommandable.

2. Statif selon la revendication 1, **caractérisé en ce que** l'on fournit pour la variation de longueur au moins un entraînement télécommandable et **en ce que** l'entraînement est disposé en dehors du montant diagonal de l'élément de support (6, 6a, 6b) et comprend un mécanisme de broche (17).

3. Statif selon l'une quelconque des revendications précédentes, **caractérisé en ce que** le premier montant diagonal (6, 6a, 6b) présente, pour la variation en longueur, au moins un moteur (11), qui est réalisé sous forme électrique, hydraulique ou pneumatique ou sous forme de moteur à ressort.

4. Statif selon l'une quelconque des revendications précédentes, **caractérisé en ce que** l'on fournit une unité de détection de déplacement/position (14) et une unité de mémoire de position qui permet un positionnement répétable dans des positions prédéfinies et **en ce que** l'unité de détection de déplacement/position (14) et/ou l'unité de mémoire de position comprennent au moins une commande de processeur (15) pour la commande automatique du déplacement ou du positionnement.

5. Statif selon l'une quelconque des revendications précédentes, **caractérisé en ce que** le deuxième montant diagonal (6, 6a, 6b) est équipé d'un élément d'amortissement.

6. Statif selon l'une quelconque des revendications précédentes, **caractérisé en ce qu'**il comprend des capteurs de déplacement, de position, d'angle ou d'oscillation (14).

7. Statif selon l'une quelconque des revendications 2 à 6, **caractérisé en ce que** le montant diagonal (6, 6a, 6b) est maintenu dans ses points d'articulation à l'intérieur de la construction porteuse avec quatre supports pouvant pivoter les uns par rapport aux autres (5a-5d) et est maintenu sans jeu par rapport à ses entraînements (11).
